# EUROPEAN PATENT SPECIFICATION

(11) **EP 0 946 175 B1**
(45) Date of publication and mention of the grant of the patent: **08.01.2003**
(21) Application number: 97945135.8
(22) Date of filing: 01.10.1997
(51) Int. Cl.: A61K 31/44, A61K 31/415, A61P 11/02

(54) **USE OF A COMBINATION OF H+, K+ -ATPase INHIBITORS AND GLUCOCORTICOIDS IN THE TREATMENT OF NASAL POLYPS**
VERWENDUNG EINER ZUSAMMENSETZUNG AUS H+, K+ -ATPase-INHIBITOREN UND GLUCOCORTIKOIDEN ZUR BEHANDLUNG VON NASENPOLYPEN
UTILISATION D'UNE COMBINAISON D' INHIBITEURS DE H+, K+ -ATPase ET DE GLUCOCORTICOIDS DANS LE TRAITEMENT DES POLYPES DU NEZ

(30) Priority: 11.10.1996 SE 9603725
(43) Date of publication of application: 06.10.1999
(73) Proprietor: AstraZeneca AB, 151 85 Södertälje (SE)
(72) Inventor: LINDBERG, Per, S-431 51 Mölndal (SE); PINAS-MASSO, Joan, E-08029 Barcelona (ES); SERRA-CARRERAS, Jordi, E-08240 Manresa (ES); TROFAST, Jan, S-226 47 Lund (SE)
(86) International application number: SE9701651
(87) International publication number: WO98016228

(56) References cited:
- US-A- 4 199 578
- US-A- 4 364 923
- J. CLIN. GASTROENTEROL., Volume 23, No. 1, 1996, M.M. YOUSFI et al., "Resolution of a Metaplastic Duodenal Polyp After Cure of Helicobacter Pylori Infection", pages 53-54.
- AMERICAN JOURNAL OF GASTROENTEROLOGY, Volume 91, No. 10, 1996, DAVID JOHNSON, "World Literature Review", pages 2245-2246.

## Description

### Field of the invention

The present invention provides a new treatment for polyposis and Widal's Syndrome using proton pump inhibitors (PPIs), i.e. H⁺, K⁺-ATPase inhibitors and glucocorticoids in simultaneous, separate or sequential administration.

### Background of the invention

Polyposis can generally arise in the nose and the gastrointestinal tract. In the nose, polyps are pale bags of tissue that arise in the nasal cavity. Their paleness is generally due to poor blood supply. It is not known what causes the polyps to be formed but their presence is often associated with certain medical conditions, for example asthma and aspirin intolerance. Within the general population the incidence of nasal polyps is low at around only 1% but 13% of asthma sufferers and 36% of aspirin intolerant asthmatics suffer from nasal polyposis. The triple condition of nasal polyposis, aspirin intolerance and asthma is known as Widal's Syndrome.

Nasal polyposis is generally treated in two stages. Initially a reduction in size of the polyps is achieved either by surgery or by the application of a topical intranasal steroid preparation, for example betamethasone sodium phosphate. Once a reduction in size has been obtained then long term maintenance of the reduction is necessary by regular use of an intranasal steroid spray such as beclomethasone dipropionate, budesonide, or fluticasone propionate. When rapid amelioration is required, oral steroids such as prednisolone or dexamethasone or synthetic adrenocorticotrophic hormones are used (see V J Lund Diagnosis and treatment of nasal polyps Brit Med J 1995, 311, 1411-4). There are also proposals that non-steroidal antiinflammatory drug can be used in the treatment of nasal polyposis (see WO 9703659-A).

### Detailed Description of the Invention

According to the invention there is provided the use of an H⁺, K⁺-ATPase inhibitor and a glucocorticoid in the manufacture of medicament for the treatment of nasal polyps which method comprises treating a subject suffering from the said condition with an H⁺, K⁺-ATPase inhibitor.

H⁺, K⁺-ATPase inhibitors are a known class of pharmaceutical agents generally used in therapy for the treatment of gastric acid related diseases. Examples of H⁺, K⁺-ATPase inhibitors are for instance compounds known under the generic names omeprazole, lansoprazole, pantoprazole, rabeprazole and leminoprazole. Some of these compounds are for instance disclosed in EP-A1-0005129, EP-A1-174726, EP-A1-166287 and GB 2163747.

These pharmaceutical substances are generally known to be useful for inhibiting gastric acid secretion in mammals and man by controlling gastric acid secretion at the final step of the acid secretory pathway. Thus, in a more general sense, they may be used for prevention and treatment of gastric-acid related diseases in mammals and man, including e.g. reflux oesophagitis, gastritis, duodenitis, gastric ulcers and duodenal ulcers.

An effect of these H⁺, K⁺-ATPase inhibitors on asthma associated with gastric reflux has been shown (see American Journal of Gastroenerology, volume 91, No 10, 1996, David Johnson, 'World Literature Review', Pages 2245-2246.)
Also the use of omeprazole for treatment of duodenal polyps has been shown in the literature. (see J. Clin. Gastroenterol., Volume 23. No. 1, 1996, M.M. Yousfi et al., 'Resolution of Metaplsatic Duodenal Polyp After Cure of Helicobacter Pylori Infection', pages 53-54).

It has now surprisingly been found that H⁺, K⁺-ATPase inhibitors in association with glucocorticoids are useful in the treatment of nasal polyps, particularly where known treatments have failed.

The H⁺, K⁺-ATPase inhibitors preferably used in the invention are compounds of the general formula 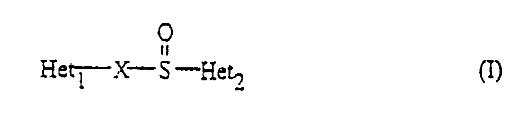 wherein
Het₁ is 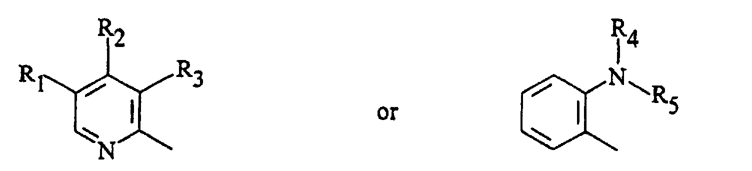
Het₂ is 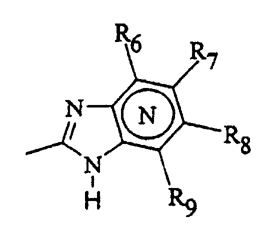
and X is 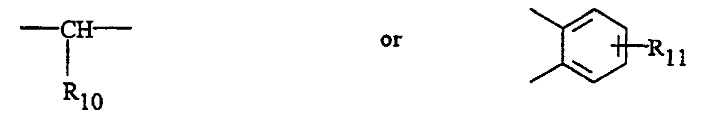
wherein
N in the benzimidazole moiety of Het₂ means that one of the ring carbon atoms substituted by R₆-R₉ optionally may be exchanged for a nitrogen atom without any substituents;
R₁ and R₃ each independently represent hydrogen, alkyl, or alkoxy on the condition that R₁ and R₃ do not simultaneously represent alkoxy; and R₂ represents alkyl, alkoxy optionally substituted by fluorine, alkylthio or alkoxyalkoxy; or one of R₁ and R₃ is halogen and the other is hydrogen and R₂ is 1-morpholino, 1-piperidino or dialkylamino;
R₄ and R₅ are the same or different and selected from hydrogen and alkyl;
R₆-R₉ are the same or different and selected from hydrogen, halogen, alkyl, alkoxy, haloalkoxy, alkylcarbonyl, and alkoxycarbonyl;
R₁₀ is hydrogen or R₁₀ and R₃ together complete a ring containing 6 to 8 carbon atoms; and
R₁₁ represents hydrogen, halogen or alkyl;
wherein the compound of formula (I) is optionally in the form of an pharmaceutically acceptable alkaline salt or in its neutral form or is a single enantiomer or a racemic mixture thereof;
wherein each alkyl or alkylenyl moiety has a branched or straight chain and has 1 to 6, preferably 1 to 4, carbon atoms;
wherein a halogen atom is preferably a fluorine, chlorine, or bromine atom, preferably a fluorine or chlorine atom.

Examples of particularly preferred compounds according to formula I for use in the invention are 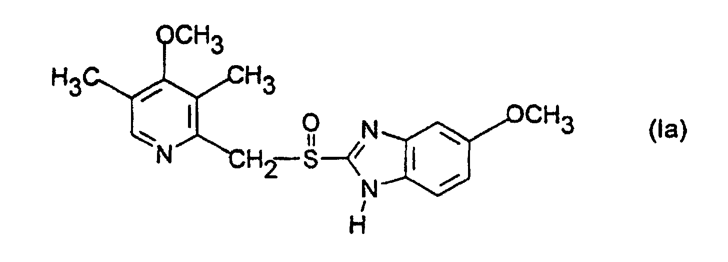 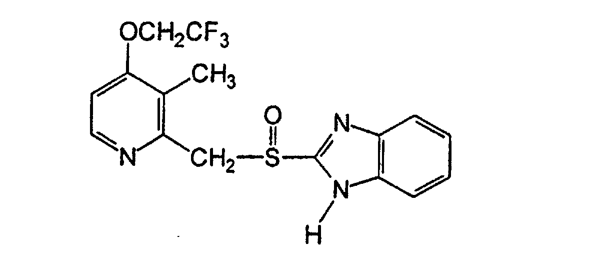 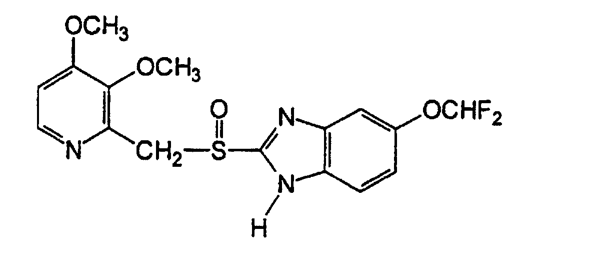 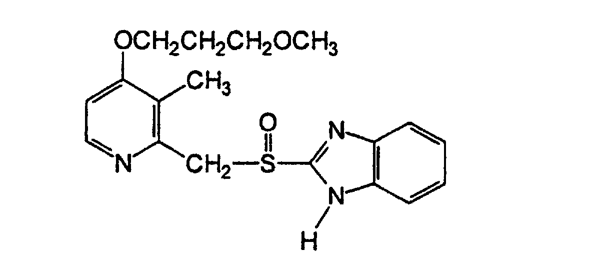 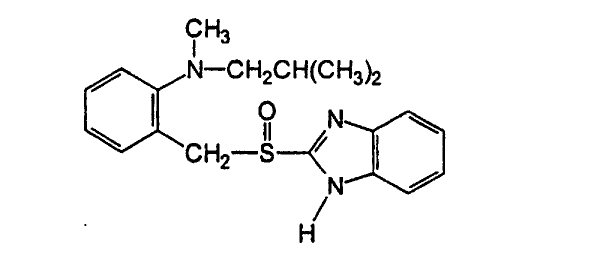 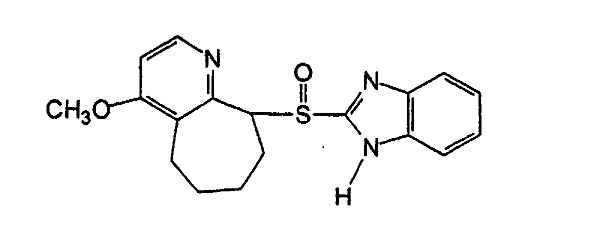 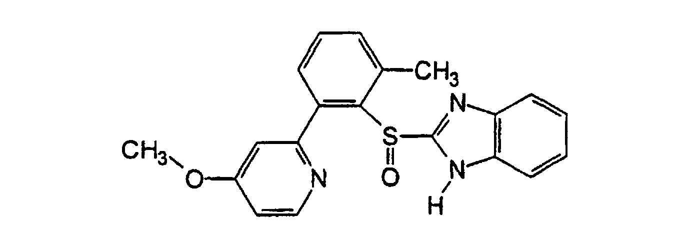 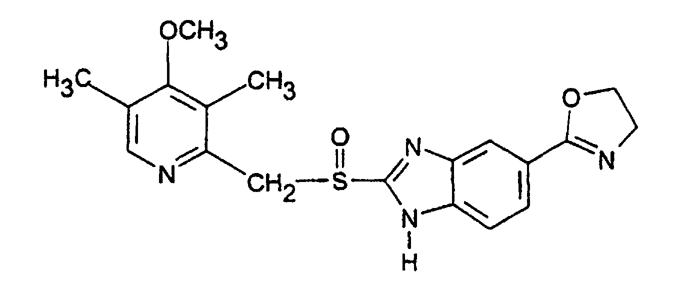 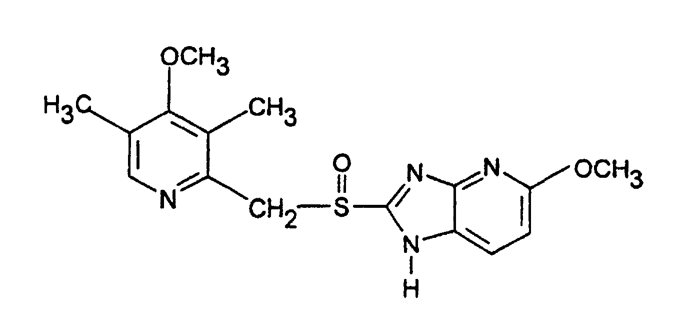 and

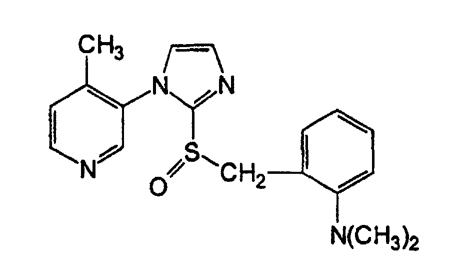

The H⁺, K⁺-ATPase inhibitor used in the invention is preferably of formula (Ia): in other words it is preferably omeprazole, or an alkaline salt of omeprazole, the (-)-enantiomer of omeprazole or an alkaline salt thereof.

The compound of formula (I) when optionally in the form of a pharmaceutically acceptable alkaline salt is preferably the Mg²⁺, Ca²⁺, Na⁺ or K⁺ salt, more preferably the Mg²⁺ salt.

The H⁺, K⁺-ATPase inhibitor and the glucocorticoid used in the invention can be administered orally, rectally or parenterally. While the effect of the inhibitors on the nasal polyps has been established in patients who have taken omeprazole by the oral route, it is believed that the effect of the inhibitor on the polyps is a systemic effect that is not dependent on what mode of administration is used. Accordingly a reduction in size of the polyps should be obtainable with other routes of administration.

Commercially available pharmaceutical preparations of H⁺, K⁺-ATPase inhibitors are suitably used in the invention. Examples of such preparations for omeprazole include enteric coated pellets of omeprazole filled in capsules, or formulated into a multiple unit tableted dosage form; enteric coated tablets of omeprazole or an alkaline salt thereof; and solutions for parenteral administration comprising an alkaline salt of omeprazole.

The dose of the H⁺, K⁺-ATPase inhibitor to be administered will vary according to the type of nasal polyps to be treated and the condition of the patient. However the dosage for oral, rectal or i.v. administration is generally in the range of from 1 to 100 mg of H⁺, K⁺-ATPase inhibitor per day. Normally an amount of from 10 to 40 mg per day is used for oral administration.

The invention may be applied in combination with other treatments known to ameliorate the other symptoms generally associated with nasal polyps. In other words, the invention can be applied in the treatment of Widal's Syndrome which consists of the conditions of nasal polyps, asthma and aspirin intolerance. According to the invention there is also provided a pharmaceutical formulation for simultaneous, separate or sequential administration to be used in the treatment of Widal's Syndrome which formulation comprises an H⁺, K⁺-ATPase inhibitor and a glucocorticoid. The invention further provides the use of an H⁺, K⁺-ATPase inhibitor and a glucocorticoid in the manufacture of such a pharmaceutical formulation.

Preferred glucocorticoids are topically active anti-inflammatory steroids. Examples of suitable steroids include budesonide; rofleponide; rofleponide palmitate; ciclesonide; momethasone furoate; fluticasone propionate; 16α, 17α-butylidenedioxy-6α,9α-difluoro-11β, 21-dihydroxypregna-1, 4-diene-3, 20-dione; 6α, 9α-difluoro-11β-hydroxy-16α, 17α-dibutylidenedioxy-17α-methylthio-androsta-4-ene-3-one; S-methyl-16α, 17α-butylidenedioxy-6α, 9α-difluoro-11β-hydroxy-3-oxo-androsta-1, 4-diene 17β-carbothioate; methyl 9α-chloro-6α-fluoro-11α-hydroxy-16α-methyl-3-oxo-17α-propionyloxy-androsta-1, 4-diene-17α-carboxylate; 6α,9α-difluoro-11β-hydroxy-16α-methyl-3-oxo-17α-propionyloxy-androsta-1,4-diene-17β-carbothioic acid S-(2-oxo-tetrahydro-furan-3S-yl) ester; tipredane; fluocinolone acetonide; flunisolide; flumethasone; dexamethasone; betamethasone; beclomethasone dipropionate; deflazacort; cortivazol; or cortisol and/or hydrocortisol, optionally in their pure isomeric forms (where such forms exist) and in the forms of their pharmaceutically acceptable salts.

The steroids for use in the invention may be applied using conventional dosing rates, e.g. 40 to 3000 µg per day. Administration may be by inhalation orally or intranasally. The steroids can optionally be adapted to be administered from a dry powder inhaler, a pressurised metered dose inhaler, or a nebuliser.

When the steroids are administered from a pressurised inhaler, they are preferably in micronised form. They are suspended or dissolved in a liquid propellant mixture. The propellants which can be used include chlorofluorocarbons, hydrocarbons or hydrofluoroalkanes. Especially preferred propellants are P134a (tetrafluoroethane) and P227 (heptafluoropropane) each of which may be used alone or in combination. They are optionally used in combination with other propellants and/or surfactants and/or other excipients, for example ethanol, surfactants, lubricants, anti-oxidants and stabilising agents.

When the steroids are administered via a nebuliser they may be in the form of a nebulised aqueous suspension or solution, with or without a suitable pH or tonicity adjustment, either as a unit dose or multidose device.

The invention is described more in detail with reference to the following examples.

### Example 1

A 53 year old woman who had had Widal's Syndrome for several years but who had refused surgical treatment of her polyps suffered mild upper abdominal pain and no improvement in the polyps after treatment by topical and systemic corticosteroids. However within two weeks of being prescribed 20 mg of omeprazole per day in addition to 100 µg of budesonide (Aqua preparation) per nostril/b.i.d. and 6 mg of deflazacort per day, she experienced a progressive improvement in her nasal respiratory problem. Eventually she recovered completely from the polyps.

### Examples 2 to 10

Nine patients, each with the conditions shown in the following Table 1, were treated during a two week period. The treatment consisted of 20 mg of omeprazole, 100 µg of intranasal budesonide and 3 to 15 mg of oral deflazacort (deflazacort was used in such a small quantity to ensure the patients' compliance). The results are also shown in Table 1.

**Table 1**

| Example No. | Condition | Result |
|---|---|---|
| 2 | Widal's Syndrome | Temporary benefit |
| 3 | Widal's Syndrome | Positive effect |
| 4 | Widal's Syndrome | Positive effect |
| 5 | Nasal Polyposis | Long term benefit |
| 6 | Nasal Polyposis | No benefit |
| 7 | Nasal Polyposis | Positive effect |
| 8 | Nasal Polyposis | Positive effect |
| 9 | Nasal Polyposis and aspirin intolerance | Positive effect |
| 10 | Nasal Polyposis and asthma | No benefit |

Where a positive effect is indicated, this means that the patient experienced a decrease in rhinorrhoea, a marked improvement in nasal respiratory ventilation and a reduction in the size of the polyps. The patient who experienced a temporary benefit by the treatment suffered a recurrence of the polyposis following the withdrawal of omeprazole and deflazacort (topical anti-inflammatory steroids, i.e. budesonide, were taken as required). However after treatment with the same regimen was resumed, a marked reduction in the size of the polyps was achieved.

The patient of Example 5 who experienced a long term benefit initially experienced no benefit at the end of the initial 2 week treatment period but continued with omeprazole and was rewarded with a positive effect at the end of 2 months.

## Claims

1. Use of an H⁺, K⁺-ATPase inhibitor and a glucocorticoid in the manufacture of a pharmaceutical formulation intended for simultaneous, separate or sequential administration in the treatment of Widal's Syndrome.

2. Use of an H⁺. K⁺-ATPase inhibitor and a glucocorticoid in the manufacture of a pharmaceutical formulation intended for simultaneous, separate or sequential administration in the treatment of nasal polyps.

3. Use according to claim 1-2 wherein the glucocorticoid is a topically active anti-inflammatory steroid.

4. Use according to claim 3 wherein the glucocorticoid is budesonide, beclomethasone dipropionate or fluticasone propionate.

5. Use according to any one of the preceding claims wherein the H⁺. K⁺-ATPase inhibitor is a compound of formula 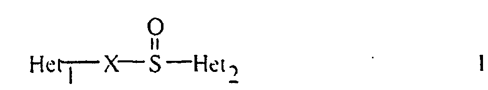 wherein
Het₁ is 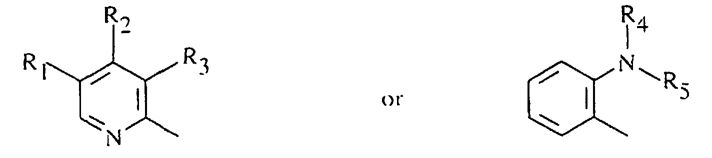
Het₂ is 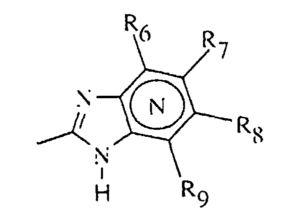
and X is 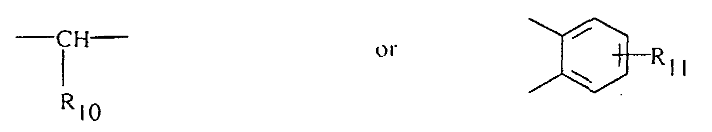
wherein
N in the benzimidazole moiety of Het₂ means that one of the ring carbon atoms substituted by R₆-R₉ optionally may be exchanged for a nitrogen atom without any substituents;
R₁ and R₃ each independently represent hydrogen, alkyl, or alkoxy on the condition that R₁ and R₃ do not simultaneously represent alkoxy: and R₂ represents alkyl, alkoxy optionally substituted by fluorine, alkylthio or alkoxyalkoxy: or one of R₁ and R₃ is halogen and the other is hydrogen and R₂ is 1-morpholino, 1-piperidino or dialkylamino;
R₄ and R₅ are the same or different and selected from hydrogen and alkyl;
R₆-R₉ are the same or different and selected from hydrogen, halogen, alkyl, alkoxy, haloalkoxy, alkylcarbonyl, and alkoxycarbonyl;
R₁₀ is hydrogen or R₁₀ and R₃ together complete a ring containing 6 to 8 carbon atoms; and
R₁₁ represents hydrogen, halogen or alkyl;
wherein the compound of formula (I) is optionally in the form of a pharmaceutically acceptable alkaline salt or in its neutral form or is a single enantiomer or a racemic mixture thereof;
wherein each alkyl or alkylenyl moiety has a branched or straight chain and has 1 to 6 carbon atoms.

6. Use according to claim 5 wherein the compound of formula (I) is a compound of formula 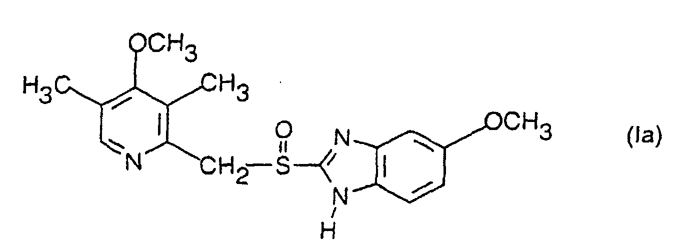 or an alkaline salt thereof, or the (-)-enantiomer or an alkaline salt of the (-)-enantiomer.

7. A pharmaceutical formulation for simultaneous, separate or sequential use in the treatment of Widal's Syndrome which formulation comprises an H⁺. K⁺-ATPase inhibitor and a glucocorticoid.

8. A pharmaceutical formulation for simultaneous, separate or sequential administration in the treatment of nasal polyps which pharmaceutical formulation comprises an H⁺, K⁺-ATPase inhibitor and a glucocorticoid.

9. A pharmaceutical formulation according to claim 7 or 8 wherein the glucocorticoid is a glucocorticoid defined in claim 3 or 4.

10. A pharmaceutical formulation according to any of claims 7 - 9 wherein the H⁺. K⁺-ATPase inhibitor is a compound of the formula defined in claim 5 or 6.

## Patentansprüche

1. Verwendung eines H⁺, K⁺-ATPase-Inhibitors und eines Glucocorticoids zur Herstellung einer pharmazeutischen Formulierung zur gleichzeitigen, getrennten oder aufeinanderfolgenden Verabreichung bei der Behandlung von Widal-Syndrom.

2. Verwendung eines H⁺, K⁺-ATPase-Inhibitors und eines Glucocorticoids zur Herstellung einer pharmazeutischen Formulierung zur gleichzeitigen, getrennten oder aufeinanderfolgenden Verabreichung bei der Behandlung von Nasenpolypen.

3. Verwendung nach Anspruch 1 oder 2, wobei es sich bei dem Glucocorticoid um ein topisch wirkendes entzündungshemmendes Steroid handelt.

4. Verwendung nach Anspruch 3, wobei es sich bei dem Glucocorticoid um Budesonid, Beclomethasondipropionat oder Fluticasonpropionat handelt.

5. Verwendung nach einem der vorhergehenden Ansprüche, wobei es sich bei dem H⁺, K⁺-ATPase-Inhibitor um eine Verbindung der Formel 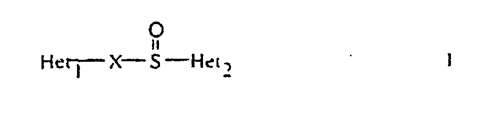 handelt, wobei
Het₁ für 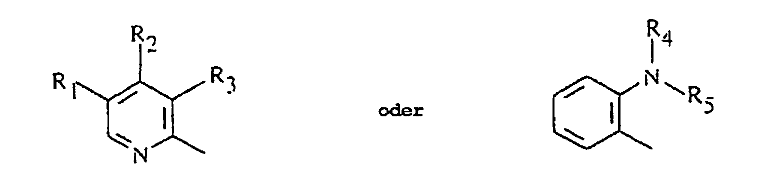 steht,
Het₂ für 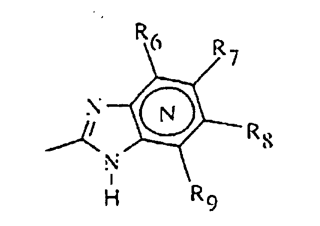 steht 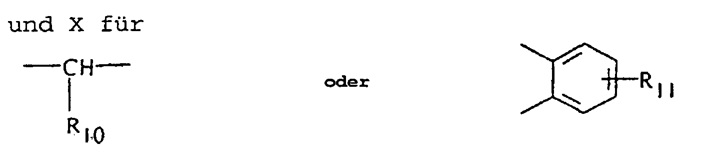 steht,
wobei
N in der Benzimidazoleinheit von Het₂ bedeutet, daß eines der durch R₆-R₉ substituierten Ringkohlenstoffatome gegebenenfalls gegen ein Stickstoffatom ohne irgendwelche Sustituenten ausgetauscht werden kann;
R₁ und R₃ jeweils unabhängig für Wasserstoff, Alkyl oder Alkoxy stehen, mit der Maßgabe, daß R₁ und R₃ nicht gleichzeitig für Alkoxy stehen; und R₂ für Alkyl, Alkoxy, gegebenenfalls substituiert durch Fluor, Alkylthio oder Alkoxyalkoxy steht, oder einer der Reste R₁ und R₃ für Halogen und der andere für Wasserstoff steht und R₂ für 1-Morpholino, 1-Piperidino oder Dialkylamino steht;
R₄ und R₅ gleich oder verschieden sind und aus Wasserstoff und Alkyl ausgewählt sind;
R₆-R₉ gleich oder verschieden sind und aus Wasserstoff, Halogen, Alkyl, Alkoxy, Halogenalkoxy, Alkylcarbonyl und Alkoxycarbonyl ausgewählt sind;
R₁₀ für Wasserstoff steht oder R₁₀ und R₃ zusammen einen Ring mit 6 bis 8 Kohlenstoffatomen bilden; und
R₁₁ für Wasserstoff, Halogen oder Alkyl steht;
wobei die Verbindung der Formel (I) gegebenenfalls in Form eines pharmazeutisch unbedenklichen alkalischen Salzes oder in seiner Neutralform vorliegt oder es sich um ein einzelnes Enantiomer oder eine racemische Mischung davon handelt;
wobei die Alkyl- und Alkylenyleinheiten jeweils verzweigt oder geradkettig sind und 1 bis 6 Kohlenstoffatome aufweisen.

6. Verwendung nach Anspruch 5, wobei es sich bei der Verbindung der Formel (I) um eine Verbindung der Formel 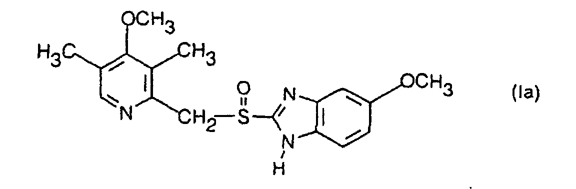 oder ein alkalisches Salz davon oder das (-)-Enantiomer oder ein alkalisches Salz des (-)-Enantiomeren handelt.

7. Pharmazeutische Formulierung zur gleichzeitigen, getrennten oder aufeinanderfolgenden Verabreichung bei der Behandlung von Widal-Syndrom, enthaltend einen H⁺, K⁺-ATPase-Inhibitor und ein Glucocorticoid.

8. Pharmazeutische Formulierung zur gleichzeitigen, getrennten oder aufeinanderfolgenden Verabreichung bei der Behandlung von Nasenpolypen, enthaltend einen H⁺, K⁺-ATPase-Inhibitor und ein Glucocorticoid.

9. Pharmazeutische Formulierung nach Anspruch 7 oder 8, wobei es sich bei dem Glucocorticoid um ein in Anspruch 3 oder 4 definiertes Glucocorticoid handelt.

10. Pharmazeutische Formulierung nach einem der Ansprüche 7-9, wobei es sich bei dem H⁺, K⁺-ATPase-Inhibitor um eine Verbindung der in Anspruch 5 oder 6 definierten Formel handelt.

## Revendications

1. Utilisation d'un inhibiteur de la H⁺, K⁺-ATPase et d'un glucocorticoïde pour l'élaboration d'une formulation pharmaceutique destinée à une administration simultanée, séparée ou séquentielle dans le traitement du syndrome de Widal.

2. Utilisation d'un inhibiteur de la H⁺, K⁺-ATPase et d'un glucocorticoïde pour l'élaboration d'une formulation pharmaceutique destinée à une administration simultanée, séparée ou séquentielle dans le traitement de polypes nasaux.

3. Utilisation selon la revendication 1-2, dans laquelle le glucocorticoïde est un stéroïde anti-inflammatoire topiquement actif.

4. Utilisation selon la revendication 3, dans laquelle le glucocorticoïde est le budésonide, le dipropionate de béclométhasone ou le propionate de fluticasone.

5. Utilisation selon l'une quelconque des revendications précédentes, dans laquelle l'inhibiteur de la H⁺, K⁺-ATPase est un composé de formule 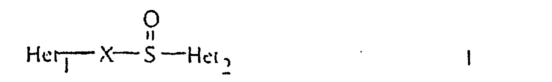 dans laquelle
Het₁ est 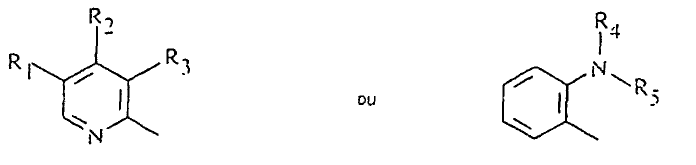
Het₂ est 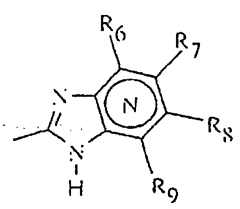
et X est 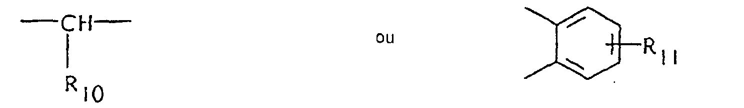
où
N dans le groupement benzimidazole de Het₂ signifie que l'un des atomes de carbone cycliques substitués par R₆-R₉ peut être éventuellement remplacé par un atome d'azote sans aucun substituant ;
R₁ et R₃ représentent chacun indépendamment un hydrogène, un alkyle ou un alcoxy à condition que R₁ et R₃ ne représentent pas simultanément un alcoxy ; et R₂ représente un alkyle, un alcoxy éventuellement substitué par un fluor, un alkylthio ou un alcoxyalcoxy ; ou l'un parmi R₁ et R₃ est un halogène et l'autre est un hydrogène et R₂ est un 1-morpholino, un 1-pipéridino ou un dialkylamino ;
R₄ et R₅ sont identiques ou différents et sont choisis parmi un hydrogène et un alkyle ;
R₆-R₉ sont identiques ou différents et sont choisis parmi un hydrogène, un halogène, un alkyle, un alcoxy, un halogénoalcoxy, un alkylcarbonyle et un alcoxycarbonyle ;
R₁₀ est un hydrogène ou R₁₀ et R₃ complètent conjointement un noyau renfermant de 6 à 8 atomes de carbone ; et
R₁₁ représente un hydrogène, un halogène ou un alkyle ;
où le composé de formule (I) est éventuellement sous la forme d'un sel alcalin pharmaceutiquement acceptable ou sous sa forme neutre ou est un seul énantiomère ou un mélange racémique de celui-ci ;
où chaque groupement alkyle ou alkylényle présente une chaîne ramifiée ou linéaire et renferme de 1 à 6 atomes de carbone.

6. Utilisation selon la revendication 5, dans laquelle le composé de formule (I) est un composé de formule 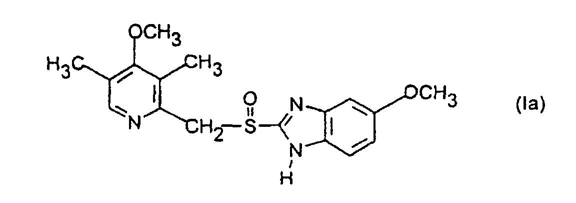 ou un sel alcalin de celui-ci, ou l'énantiomère (-) ou un sel alcalin de l'énantiomère (-).

7. Formulation pharmaceutique destinée à une utilisation simultanée, séparée ou séquentielle dans le traitement du syndrome de Widal, laquelle formulation comprend un inhibiteur de la H⁺, K⁺-ATPase et un glucocorticoïde.

8. Formulation pharmaceutique destinée à une administration simultanée, séparée ou séquentielle dans le traitement de polypes nasaux, laquelle formulation pharmaceutique comprend un inhibiteur de la H⁺, K⁺-ATPase et un glucocorticoïde.

9. Formulation pharmaceutique selon la revendication 7 ou 8, dans laquelle le glucocorticoïde est un glucocorticoïde défini dans la revendication 3 ou 4.

10. Formulation pharmaceutique selon l'une quelconque des revendications 7 à 9, dans laquelle l'inhibiteur de la H⁺, K⁺-ATPase est un composé de formule définie dans la revendication 5 ou 6.
